# EUROPEAN PATENT APPLICATION

(11) **EP 4 052 713 A1**
(43) Date of publication of application: **07.09.2022**
(21) Application number: 20883328.5
(22) Date of filing: 30.10.2020
(51) Int. Cl.: A61K 31/78, A61K 47/58, C07D 403/12, C07D 401/12, C07D 405/14, C07D 401/14, A61P 35/00

(54) **PHARMACEUTICAL COMPOSITION COMPRISING QUINAZOLINE DERIVATIVE OR SALT THEREOF**

(30) Priority: 01.11.2019 CN 201911059476
(71) Applicant: CHIA TAI TIANQING PHARMACEUTICAL GROUP CO., LTD., Lianyungang, Jiangsu 222062 (CN); Lianyungang Runzhong Pharmaceutical Co., Ltd., Lianyungang, Jiangsu 222069 (CN); Shouyao Holdings (Beijing) Co., Ltd., Beijing 100176 (CN)
(72) Inventor: SHANG, Lei, Lianyungang, Jiangsu 222062 (CN); HE, Jiali, Lianyungang, Jiangsu 222062 (CN); YAN, Caixia, Lianyungang, Jiangsu 222062 (CN); DONG, Ping, Lianyungang, Jiangsu 222062 (CN); ZHOU, Jie, Lianyungang, Jiangsu 222062 (CN); SUN, Yingying, Lianyungang, Jiangsu 222062 (CN); XU, Yi, Lianyungang, Jiangsu 222062 (CN); CHEN, Zhilin, Lianyungang, Jiangsu 222062 (CN); TANG, Song, Lianyungang, Jiangsu 222062 (CN)
(74) Representative: Potter Clarkson
(86) International application number: PCT/CN2020/125383
(87) International publication number: WO 2021/083346

(57) **Abstract**

A solid pharmaceutical composition comprising a quinazoline derivative or a medicinal salt thereof, and a preparation method therefor. Specifically, provided is a solid pharmaceutical composition comprising N⁶-(1-acryloylpiperidin-4-yl)-N⁴-(3-chloro-4-fluorophenyl)-7-methoxyquinazoline-4,6-diamine or a medicinal salt thereof, and a preparation method therefor and use thereof.

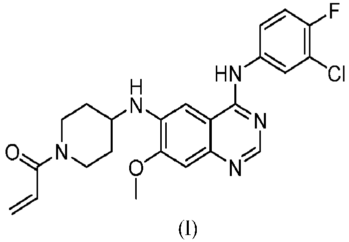

## Description

### CROSS-REFERENCE TO RELATED APPLICATIONS

The present application claims priority to and benefit of the Chinese Patent Application No. 201911059476.1 filed with National Intellectual Property Administration, PRC on Nov. 1, 2019, the disclosure of which is incorporated herein by reference in its entirety.

### TECHNICAL FIELD

The present application belongs to the technical field of pharmaceuticals, and relates to a pharmaceutical composition comprising a quinazoline derivative or a pharmaceutically acceptable salt thereof and a preparation method thereof, and particularly to a pharmaceutical composition comprising N⁶-(1-acryloylazacyclohexane-4-yl)-N⁴-(3-chloro-4-fluorophenyl)-7-methoxyquinazoline-4,6-diamine or a pharmaceutically acceptable salt thereof, a preparation method thereof and use thereof.

### BACKGROUND

EGFR is a tyrosine kinase receptor and is a member of the HER/ErbB family, which comprises EGFR, HER2, HER3 and HER4. It consists of three parts: an extracellular ligand-binding domain, a transmembrane domain composed of single chains and an intracellular tyrosine kinase domain. EGFR is widely distributed on the surface of mammalian cells such as epithelial cells, fibroblasts, glial cells and keratinocytes. The EGFR signaling pathway plays an important role in physiological processes such as growth, proliferation and differentiation of cells. The functional deficiency of protein tyrosine kinases such as EGFR, the abnormal activity of key factors in related signaling pathways, or abnormal cellular localization will lead to the occurrence of tumors, diabetes, immunodeficiency and cardiovascular diseases.

The compound of formula **(I)** disclosed in WO2015043515A is a selective epidermal growth factor receptor inhibitor. It can competitively bind to intracellular tyrosine kinases at the phosphorylation sites, blocking the interaction between it and ATP, inhibiting the phosphorylation of tyrosine and a series of downstream signal transduction and thus inhibiting the growth of tumor cells. It can be used to treat a variety of malignant tumors such as non-small cell lung cancer and breast cancer. The chemical name of the compound of formula (I) is N⁶-(1-acryloylazacyclohexane-4-yl)-N⁴-(3-chloro-4-fluorophenyl)-7-methoxyquinazoline-4,6-diamine.

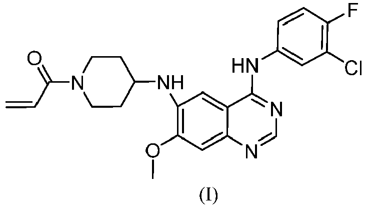

When the compound of formula (I) and a pharmaceutically acceptable salt thereof of the present application are prepared into oral capsule formulations, the active ingredients are susceptible to reactions to form impurities during storage; the increasing of the impurities is difficult to control, and thus the formulations can hardly be stored stably for a long period of time. Due to the fact that whether the amount of impurities in a medicament can be reasonably and effectively controlled is directly associated with the quality controllability and safety of the medicament, it is necessary to solve the problem described above.

### SUMMARY

In one aspect, the present application provides a pharmaceutical composition comprising a compound of formula (I) or a pharmaceutically acceptable salt thereof, and a polymethacrylate ester

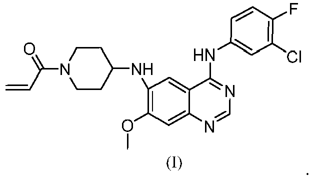

In some embodiments, the pharmaceutical composition described above is a solid pharmaceutical composition.

In some embodiments, the pharmaceutically acceptable salt described above is selected from the group consisting of a maleate salt, a hydrochloride salt, a hydrobromide salt, a sulfate salt, a phosphate salt, a nitrate salt, an acetate salt, a lactate salt, a malonate salt, a succinate salt, a fumarate salt, a malate salt, a mandelate salt, a tartrate salt, a citrate salt, an ascorbate salt, a palmitate salt, a benzoate salt, a phenylacetate salt, a cinnamate salt, a salicylate salt, a methanesulfonate salt, a benzenesulfonate salt, and a methylbenzenesulfonate salt.

In some embodiments, the pharmaceutically acceptable salt described above is selected from the group consisting of a maleate salt, a malate salt, a fumarate salt, a tartrate salt, a citrate salt, a lactate salt, a phosphate salt, and an acetate salt.

In some embodiments, the pharmaceutically acceptable salt described above is selected from a maleate salt.

In some embodiments, a molar ratio of the compound of formula (I) to an acid radical ion in the pharmaceutically acceptable salt of the compound of formula (I) described above may be 1:1.

In some embodiments, in the solid pharmaceutical composition described above, the compound of formula (I) or the pharmaceutically acceptable salt thereof is selected from the group consisting of a compound of formula (I) and the maleate salt of the compound of formula I.

In some embodiments, the pharmaceutically acceptable salt of the compound of formula (I) described above is a compound of formula (II),

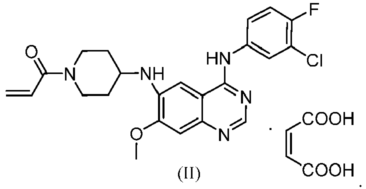

The "compound of formula (I) or the pharmaceutically acceptable salt thereof" described in the present application may be a "compound of formula (II)".

In some embodiments, the solid pharmaceutical composition described above comprises a compound of formula (II), and a polymethacrylate ester.

In some embodiments, the polymethacrylate ester described above is Eudragit^{®}.

In some embodiments, the polymethacrylate ester described above is selected from the group consisting of Eudragit^{®} E100, Eudragit^{®} EPO, Eudragit^{®} S100, Eudragit^{®} L100, Eudragit^{®} E100-55, Eudragit^{®} FS30D, Eudragit^{®} NM30D, Eudragit^{®} NE30D, Eudragit^{®} RL100, Eudragit^{®} RS100, and combinations thereof.

In some embodiments, the polymethacrylate ester described above is selected from Eudragit^{®} E100.

In some embodiments, in the solid pharmaceutical composition described above, a weight ratio of the polymethacrylate ester to the compound of formula (I) or the pharmaceutically acceptable salt thereof is (50-0.5):1, or (45-1):1, or (40-1.5):1, or (35-2):1, or (30-3):1, or (25-4):1, or (20-5):1, or (18-5.5):1, or (16-5.7): 1, or (14-5.9):1, or (12-6.1):1, or (10-6.3): 1, or (8.5-6.5): 1, or (8.5-7): 1, or (8.5-7.5): 1.

In some embodiments, in the solid pharmaceutical composition described above, a weight ratio of the polymethacrylate ester to the compound of formula (II) is 8:1.

In some embodiments, the solid pharmaceutical composition described above further comprises an acid.

In some embodiments, the acid described above is selected from the group consisting of maleic acid, malic acid, fumaric acid, tartaric acid, citric acid, lactic acid, phosphoric acid, acetic acid, and combinations thereof.

In some embodiments, the acid described above is maleic acid.

In some embodiments, the solid pharmaceutical composition described above comprises 0.1-50 wt%, or 0.1-20 wt%, or 0.1-10 wt%, or 0.1-5 wt%, or 0.1-4 wt% of the compound of formula (I) or the pharmaceutically acceptable salt thereof based on the total mass of the solid pharmaceutical composition.

In some embodiments, the solid pharmaceutical composition further comprises a surface stabilizer.

In some embodiments, the surface stabilizer described above is selected from the group consisting of hydroxypropylcellulose, lecithin (phospholipids), glycerol monostearate, polyoxyethylene castor oil derivatives, polyoxyethylene sorbitan fatty acid esters (e.g., commercially available tweens such as Tween 20 and Tween 80 (ICI Speciality Chemicals)), polyethylene glycols (e.g., Carbowaxs 3550 and 934 (Union Carbide)), sodium dodecyl sulfate, carboxymethylcellulose calcium, carboxymethylcellulose sodium, methylcellulose, hydroxyethylcellulose, hydroxypropylmethylcellulose, hydroxypropylmethylcellulose phthalate, amorphous cellulose, magnesium aluminum silicate, triethanolamine, polyvinyl alcohol (PVA), polyvinylpyrrolidone (PVP), 4-(1,1,3,3-tetramethylbutyl)phenol polymers with ethylene oxide and formaldehyde (also referred to as tyloxapol or triton), and poloxamers (e.g., Pluromics F68 and F108, block copolymers of ethylene oxide and propylene oxide).

In some embodiments, the surface stabilizer described above is selected from the group consisting of hydroxypropylcellulose, carboxymethylcellulose calcium, carboxymethylcellulose sodium, methylcellulose, hydroxyethylcellulose, hydroxypropylmethylcellulose, hydroxypropylmethylcellulose phthalate, and amorphous cellulose.

In some embodiments, the surface stabilizer described above is selected from hydroxypropylcellulose (e.g., an HPC-SL type).

In some embodiments, in the solid pharmaceutical composition described above, a weight ratio of the surface stabilizer to the compound of formula (I) or the pharmaceutically acceptable salt thereof is (0.06-1):1, or (0.07-0.9):1, or (0.08-0.8):1, or (0.09-0.7):1, or (0.10-0.6):1, or (0.11-0.5):1, or (0.12-0.7):1, or (0.13-0.6):1, or (0.14-0.5):1, or (0.15-0.4):1, or (0.15-0.3):1, or (0.15-0.2):1.

In some embodiments, in the solid pharmaceutical composition described above, a weight ratio of the surface stabilizer to the compound of formula (II) is 0.16:1.

Most of these surface stabilizers are known pharmaceutical excipients detailed in Handbook of Pharmaceutical Excipients published jointly by the American Pharmaceutical Association and The Pharmaceutical Society of Great Britain (the Pharmaceutical Press, 1986).

In some embodiments, the solid pharmaceutical composition further comprises a dispersant.

In some embodiments, the dispersant described above is selected from the group consisting of sucrose, lactose, and mannitol.

In some embodiments, the dispersant described above is selected from sucrose.

In some embodiments, in the solid pharmaceutical composition described above, a weight ratio of the dispersant to the compound of formula (I) or the pharmaceutically acceptable salt thereof is (0.5-50):1, or (0.8-45):1, or (1.1-40):1, or (1.4-35):1, or (1.7-30):1, or (2-25):1, or (2.3-20):1, or (2.8-15):1, or (3.1-10):1, or (3.4-9):1, or (3.7-8):1, or (4.0-7):1, or (4.3-6):1, or (4.5-5.5):1.

In some embodiments, in the solid pharmaceutical composition described above, a weight ratio of the dispersant to the compound of formula (II) is 5:1.

In some embodiments, the solid pharmaceutical composition further comprises a carrier.

In some embodiments, the carrier described above is selected from the group consisting of a cellulose sphere, a mannitol pellet core, a tartaric acid pellet core, a lactose/microcrystalline pellet core, a sucrose pellet core, a starch pellet core, and combinations thereof. In some embodiments, the carrier described above is selected from the group consisting of a cellulose sphere and a sucrose pellet core.

In some embodiments, the carrier described above is selected from a sucrose pellet core (e.g., 0.6-0.8 mm).

In some embodiments, a proportion range of the carrier in the solid pharmaceutical composition is selected from the group consisting of 0.1-99 wt%, 0.5-99 wt%, 1-99 wt%, 5-99 wt%, 10-99 wt%, 15-99 wt%, 20-99 wt%, 25-99 wt%, 30-99 wt%, 35-99 wt%, 40-99 wt%, and 45-99 wt%.

In another aspect, the present application provides a solid pharmaceutical composition comprising a compound of formula (I) or a pharmaceutically acceptable salt thereof, a polymethacrylate ester, an acid, a surface stabilizer, a dispersant, and a carrier.

In some embodiments, the pharmaceutically acceptable salt of the compound of formula (I) described above is selected from the maleate salt of the compound of formula (I), wherein a molar ratio of the compound of formula (I) to the acid radical ion involved in the formation of the maleate salt is 1:1.

In some embodiments, the solid pharmaceutical composition described above comprises a compound of formula (II), a polymethacrylate ester, an acid, a surface stabilizer, a dispersant, and a carrier,

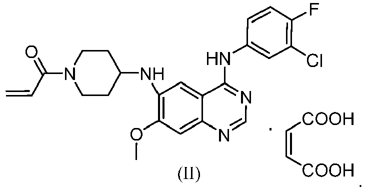

In some embodiments, in the solid pharmaceutical composition described above, the polymethacrylate ester, the acid, the surface stabilizer, the dispersant, and the carrier are each as defined above.

In some embodiments, in the solid pharmaceutical composition described above, weight content ranges or related proportions of the polymethacrylate ester, the acid, the surface stabilizer, the dispersant and the carrier are each as defined above.

In a further aspect, the present application provides a solid pharmaceutical composition comprising a compound of formula (II), hydroxypropylcellulose (e.g., an HPC-SL type), sucrose, a polymethacrylate ester (e.g., Eudragit E100), maleic acid, and a sucrose pellet core (e.g., 0.6-0.8 mm).

In some embodiments, in the solid pharmaceutical composition described above, weight content ranges or related proportions of the hydroxypropylcellulose, sucrose, polymethacrylate ester (e.g., Eudragit^{®} E100), maleic acid and sucrose pellet core are each as defined above.

The solid pharmaceutical composition described above comprises the following components in parts by weight:

| | |
|---|---|
| the compound of formula (II) | 0.1-40 parts |
| the surface stabilizer | 0.01-10 parts |
| the dispersant | 0.5-350 parts |
| the polymethacrylate ester (e.g., Eudragit^{®} E100) | 0.5-500 parts |
| the acid | |
| the carrier | 100-500 parts. |

In some embodiments, in the solid pharmaceutical composition described above, the polymethacrylate ester, the acid, the surface stabilizer, the dispersant, and the carrier are each as defined above.

The solid pharmaceutical composition described above comprises the following components in parts by weight:

| | |
|---|---|
| the compound of formula (II) | 0.1-40 parts |
| hydroxypropylcellulose (e.g., an HPC-SL type) | 0.01-10 parts |
| sucrose | 0.5-350 parts |
| the polymethacrylate ester (e.g., Eudragit^{®} E100) | 0.5-500 parts |
| maleic acid | |
| the sucrose pellet core (e.g., 0.6-0.8 mm) | 100-500 parts. |

The solid pharmaceutical composition described above comprises the following components in parts by weight:

| | |
|---|---|
| the compound of formula (II) | 0.627 parts |
| hydroxypropylcellulose (e.g., an HPC-SL type) | 0.1 parts |
| sucrose | 3.13 parts |
| the polymethacrylate ester (e.g., Eudragit^{®} E100) maleic acid | 5 parts |
| the sucrose pellet core (e.g., 0.6-0.8 mm) | 333 parts. |

The solid pharmaceutical composition described above comprises the following components in parts by weight:

| | |
|---|---|
| the compound of formula (II) | 2.508 parts |
| hydroxypropylcellulose (e.g., an HPC-SL type) | 0.4 parts |
| sucrose | 12.54 parts |
| the polymethacrylate ester (e.g., Eudragit^{®} E100) maleic acid | 20 parts |
| the sucrose pellet core (e.g., 0.6-0.8 mm) | 250 parts. |

The solid pharmaceutical composition described above comprises the following components in parts by weight:

| | |
|---|---|
| the compound of formula (II) | 6.27 parts |
| hydroxypropylcellulose (e.g., an HPC-SL type) | 1 part |
| sucrose | 31.35 parts |
| the polymethacrylate ester (e.g., Eudragit^{®} E100) maleic acid | 50 parts |
| the sucrose pellet core (e.g., 0.6-0.8 mm) | 250 parts. |

The solid pharmaceutical composition described above comprises the following components in parts by weight:

| | |
|---|---|
| the compound of formula (II) | 12.54 parts |
| hydroxypropylcellulose (e.g., an HPC-SL type) | 2 parts |
| sucrose | 62.7 parts |
| the polymethacrylate ester (e.g., Eudragit^{®} E100) maleic acid | 100 parts |
| the sucrose pellet core (e.g., 0.6-0.8 mm) | 250 parts. |

Each of the solid pharmaceutical compositions of the present application described above may further comprise compound A (impurity)

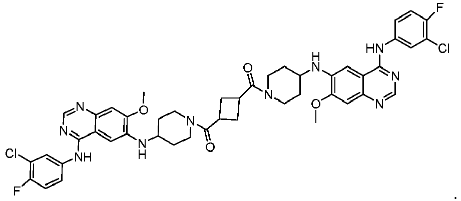

In some embodiments, compound A has a retention time of 28-30 min in HPLC analysis, wherein conditions for the HPLC analysis are as follows:
mobile phase A: 0.01 mol/L ammonium formate buffer (dissolving 0.63 g of ammonium formate in 1000 mL of water, adding 1 mL of formic acid, and adjusting the pH value to 7.4 with triethylamine);
mobile phase B: acetonitrile;
flow rate: 1.0 mL/min; wavelength: 260 nm; and column temperature: 40 °C.
Linear gradient elution is performed according to the table below.

| Time (min) | Mobile phase A (%) | Mobile phase B (%) |
|---|---|---|
| 0 | 77 | 23 |
| 50 | 20 | 80 |
| 51 | 77 | 23 |
| 60 | 77 | 23 |

In some embodiments, compound A has a relative retention time RRT of 1.3 in HPLC analysis under the conditions described above with the compound of formula (I) as a reference.

In some embodiments, in the HPLC analysis of compound A, the instrument employed for analysis is SHIMADAZU LC-20AD HPLC, and the chromatography column is Waters XBridge C18(4.6 × 150 mm, 5µm). In some embodiments, compound A has the following molecular ion peak in a mass spectrum: m/z 456.1600 ([M+2H]++).

In some embodiments, a proportion range of compound A in the solid pharmaceutical composition is selected from the group consisting of less than 10 wt%, less than 5 wt%, less than 4 wt%, less than 3 wt%, less than 1 wt%, less than 0.5 wt%, less than 0.3 wt%, and less than 0.2 wt%. In some embodiments, a content of compound A is less than 10%, or less than 5%, or less than 4%, or less than 3%, or less than 1%, or less than 0.5%, or less than 0.3%, or less than 0.2%.

In some embodiments, the solid pharmaceutical composition described above has a change that is less than 0.8%, or less than 0.6%, or less than 0.5%, or less than 0.4%, or less than 0.3%, or less than 0.2%, or less than 0.15% in the weight or content of compound A after 6 months under the conditions of a temperature of 40 °C ± 2 °C and a relative humidity of 75%± 5%.

In some embodiments, the solid pharmaceutical composition described above has a change that is less than 0.3%, or less than 0.2%, or less than 0.15%, or less than 0.1% in the weight or content of compound A after 6 months under the conditions of a temperature of 30 °C ± 2 °C and a relative humidity of 65% ± 5%.

In some embodiments, the solid pharmaceutical composition described above has a change that is less than 0.15%, or less than 0.1%, or less than 0.08%, or less than 0.05% in the weight or content of compound A after 6 months under the conditions of a temperature of 25 °C ± 2 °C and a relative humidity of 60% ± 5%.

In some embodiments, the solid pharmaceutical composition described above has a change that is less than 0.1%, or less than 0.09%, or less than 0.07%, or less than 0.05% in the weight or content of compound A after 6 months under the conditions of a temperature of 20 °C ± 2 °C and a relative humidity of 60% ± 5%.

In some embodiments, a content of compound A is determined by HPLC analysis (e.g. calculated using the principal component self-contrast method), e.g. by HPLC under the analysis conditions described above.

Particle size ranges of the compound of formula (I) or the pharmaceutically acceptable salt thereof in the solid pharmaceutical compositions of the present application described above are D50 < 10 µm and D90 < 20 µm, or D50 < 5 µm and D90 < 10 µm, or D50 < 2 µm and D90 < 4 µm.

In some embodiments, particle size ranges of the compound of formula (I) or the pharmaceutically acceptable salt thereof are D50 < 2 µm and D90 < 4 µm.

In the solid pharmaceutical compositions of the present application described above, the acid is added in a proper amount, for example, to make the solid pharmaceutical composition acidic in water.

In some embodiments, in the solid pharmaceutical compositions described above, the maleic acid is added in a proper amount.

In some embodiments, the amount of the acid used in the solid pharmaceutical compositions described above depends on the pH range required for the preparation of the solid composition, e.g., acidity in aqueous solution, more specifically, e.g., a pH value between 2.0 and 3.5 (e.g., 2.0 ≤ pH < 3.0 or 3.0 ≤ pH ≤ 3.5) in aqueous solution.

In some embodiments, in the solid pharmaceutical compositions described above, the acid can serve as a pH adjusting agent, e.g., for adjusting the pH range in the preparation of the solid pharmaceutical composition, e.g., to acidity in aqueous solution, more specifically, e.g., to a pH value between 2.0 and 3.5 (e.g., 2.0 ≤ pH < 3.0 or 3.0 ≤ pH ≤ 3.5) in aqueous solution.

The solid pharmaceutical compositions of the present application described above may be in a variety of dosage forms suitable for oral administration to a patient (e.g., a human), including, for example, tablets, pills, capsules, powders, and granules.

In some embodiments, the solid pharmaceutical compositions of the present application are orally administered. In some embodiments, the solid pharmaceutical compositions of the present application are formulated in the form of a capsule.

In some embodiments, the capsule described above is filled with pellet particles comprising the solid pharmaceutical composition described above.

In some embodiments, the capsule described above is a hard capsule or a soft capsule.

Capsules can be prepared according to known methods by filling pellet particles of the compound of formula (I) or the pharmaceutically acceptable salt thereof described above into hard capsules made of gelatin, hydroxypropylmethylcellulose, polyvinyl alcohol, or the like, or into soft capsules based on gelatin.

In yet another aspect, the present application provides a method for preparing a solid composition comprising the following steps: preparing a suspension containing a compound of formula (I) or a pharmaceutically acceptable salt thereof; adding an acid and a polymethacrylate ester to the suspension; and loading the resulting suspension on a carrier on a fluidized bed to obtain drug-containing pellets.

In another aspect, the present application provides a method for preparing a solid composition comprising the following steps:
preparing a suspension containing a compound of formula (I) or a pharmaceutically acceptable salt thereof;
adding an acid and a polymethacrylate ester to the suspension; and subjecting the suspension to fluidized bed granulation.

In some embodiments, the fluidized bed granulation comprises loading the resulting suspension on a carrier on a fluidized bed to obtain drug-containing pellets. In some embodiments, the method for preparing a solid composition described above comprises the following steps:
1) adding a surface stabilizer to a dispersion medium, adding a compound of formula (I) or a pharmaceutically acceptable salt thereof after complete dissolution, sieving the mixture after uniform dispersion, and adding a dispersant after high-pressure homogenization to obtain a suspension containing the compound of formula (I) or the pharmaceutically acceptable salt thereof;
2) preparing an acid solution, adding a polymethacrylate ester to a portion of the acid solution, after complete dissolution, adding the resulting solution to the suspension obtained in step 1), and adjusting the suspension to a pH value between 2.0 and 3.5 (e.g., 2.0 ≤ pH < 3.0 or 3.0 ≤ pH ≤ 3.5) with the rest of the acid solution to obtain a suspension; and
3) subjecting the suspension obtained in step 2) to fluidized bed granulation on a carrier.

In some embodiments, in the preparation method described above, step 1) is as follows: adding a surface stabilizer to a dispersion medium, adding a compound of formula II after complete dissolution, sieving the mixture after uniform dispersion, and adding a dispersant after high-pressure homogenization to obtain a suspension containing the compound of formula (II).

In some embodiments, in the preparation method described above, step 2) is as follows: preparing an acid solution, adding a polymethacrylate ester to a portion of the acid solution, after complete dissolution, adding the resulting solution to the suspension obtained in step 1), and adjusting the pH value of the suspension to 2.0 ≤ pH < 3.0 with the rest of the acid solution to obtain a suspension.

In some embodiments, in the preparation method described above, step 2) is as follows: preparing an acid solution, adding a polymethacrylate ester to a portion of the acid solution, after complete dissolution, adding the resulting solution to the suspension obtained in step 1), and adjusting the pH value of the suspension to a pH value between 3.0 and 3.5 with the rest of the acid solution to obtain a suspension.

In some embodiments, in the preparation method described above, the surface stabilizer, the dispersant, the acid, the polymethacrylate ester and the carrier are each as defined above.

In some embodiments, in the preparation method described above, the solid pharmaceutical composition obtained comprises 0.1-50 wt%, or 0.1-20 wt%, or 0.1-10 wt%, or 0.1-5 wt%, or 0.1-4 wt% of the compound of formula (I) or the pharmaceutically acceptable salt thereof based on the total mass of the solid pharmaceutical composition.

In some embodiments, in the preparation method described above, the dispersion medium is selected from the group consisting of methanol, ethanol, isopropanol, acetone and water.

In some embodiments, in the preparation method described above, the dispersion medium is selected from water. In some embodiments, in the preparation method described above, a mass ratio of the dispersion medium to the surface stabilizer is selected from the group consisting of (1000-300):1, (1000-500):1, (800-500):1, (700-550):1, (700-600):1, (700-650):1 and 666.7:1.

In some embodiments, in the preparation method described above, a weight ratio of the surface stabilizer to the compound of formula (I) or the pharmaceutically acceptable salt thereof is (0.06-1):1, or (0.07-0.9):1, or (0.08-0.8):1, or (0.09-0.7):1, or (0.10-0.6):1, or (0.11-0.5):1, or (0.12-0.7):1, or (0.13-0.6):1, or (0.14-0.5):1, or (0.15-0.4):1, or (0.15-0.3):1, or (0.15-0.2):1.

In some embodiments, in the preparation method described above, a weight ratio of the surface stabilizer to the compound of formula (II) is 0.16:1.

In some embodiments, in the preparation method described above, a weight ratio of the dispersant to the compound of formula (I) or the pharmaceutically acceptable salt thereof is (0.5-50):1, or (0.8-45):1, or (1.1-40):1, or (1.4-35):1, or (1.7-30):1, or (2-25):1, or (2.3-20):1, or (2.8-15):1, or (3.1-10):1, or (3.4-9):1, or (3.7-8):1, or (4.0-7):1, or (4.3-6):1, or (4.5-5.5):1.

In some embodiments, in the preparation method described above, a weight ratio of the dispersant to the compound of formula (II) is 5:1.

In some embodiments, in the preparation method described above, a weight ratio of the polymethacrylate ester to the compound of formula (I) or the pharmaceutically acceptable salt thereof is (50-0.5):1, or (45-1):1, or (40-1.5):1, or (35-2):1, or (30-3):1, or (25-4):1, or (20-5):1, or (18-5.5):1, or (16-5.7):1, or (14-5.9):1, or (12-6.1):1, or (10-6.3):1, or (8.5-6.5):1, or (8.5-7):1, or (8.5-7.5):1.

In some embodiments, in the preparation method described above, a weight ratio of the polymethacrylate ester to the compound of formula (II) is 8:1.

In some embodiments, in the preparation method described above, the acid solution is prepared with methanol, ethanol, isopropanol, acetone or water.

In some embodiments, in the preparation method described above, the acid solution is prepared with water.

In some embodiments, in the preparation method described above, the acid solution is prepared at a concentration of 0.1-2 mol/L, or 0.2-1 mol/L, or 0.3-0.8 mol/L, or 0.4-0.6 mol/L, or 0.5 mol/L with water. In some embodiments, in the preparation method described above, after high-pressure homogenization, a particle size of the drug is controlled at: D50 < 10 µm and D90 < 20 µm; or D50 < 5 µm and D90 < 10 µm; or D50 < 2 µm and D90 < 4 µm.

In some embodiments, in the preparation method described above, a range of the carrier in the solid pharmaceutical composition is selected from the group consisting of 0.1-99 wt%, 0.5-99 wt%, 1-99 wt%, 5-99 wt%, 10-99 wt%, 15-99 wt%, 20-99 wt%, 25-99 wt%, 30-99 wt%, 35-99 wt%, 40-99 wt%, and 45-99 wt%.

In some embodiments, in the preparation method described above, the solid pharmaceutical composition obtained is in the form of pellets; the preparation method optionally comprises a step of mixing well in a hopper mixer.

In some embodiments, the preparation method described above further comprises a step of filling capsules.

In another aspect, the present application provides use of the pharmaceutical composition described above for preparing a medicament for treating a tumor.

In another aspect, the present application provides a method for treating a tumor comprising administering to an individual in need a therapeutically effective amount of the pharmaceutical composition described above.

In another aspect, the present application provides the pharmaceutical composition described above for treating a tumor.

In another aspect, the present application provides use of the pharmaceutical composition described above for treating a tumor.

In some embodiments, the tumor is selected from the group consisting of: non-small cell lung cancer and breast cancer.

The solid pharmaceutical composition comprising the compound of the formula (I) and the pharmaceutically acceptable salt thereof provided in the present application has the advantages of having a low maximum single impurity content and a low total impurity content, showing no significant change in the content of active ingredients and impurities, particularly enabling effective control of the increase of compound A (impurity), having good stability, high bioavailability and a high dissolution rate, and being suitable for industrial production, storage and clinical use.

### Definitions and Description

Unless otherwise required in the present application, the word "comprise" and variations thereof such as "comprises" and "comprising", used in the specification and claims that follow, should be understood in an open-ended and inclusive sense, i.e., "including, but not limited to".

The term "optional" or "optionally" means that the subsequently described event or circumstance may, but not necessarily, occur. The description includes instances where the event or circumstance occurs and instances where the event or circumstance does not occur. It means an alternative, and is used interchangeably with "or".

"One embodiment", "an embodiment", "in another embodiment" or "in some embodiments" used in the specification means that a specific reference element, structure or characteristic described in connection with the embodiment is included in at least one embodiment. Thus, the phrases "in one embodiment", "in an embodiment", "in another embodiment" and "in some embodiments" in various places throughout the specification are not necessarily all referring to the same embodiment. Furthermore, the specific elements, structures, or characteristics may be combined in any suitable manner in one or more embodiments.

It should be understood that, unless otherwise specified clearly, the singular forms "a," "an," and "the" used in the specification and the appended claims of the present application include plural referents. Thus, for example, the mentioned reaction including "a catalyst" includes one catalyst, or two or more catalysts. It should be understood that, unless otherwise specified clearly, the term "or" is generally employed in its sense including "and/or".

The term "treat" or "treatment" means administering the compound or formulation described in the present application to ameliorate or eliminate a disease or one or more symptoms associated with the disease, and includes:
(i) inhibiting a disease or disease state, i.e., arresting its development; and
(ii) alleviating a disease or disease state, i.e., causing its regression.

The term "prevent" or "prevention" means administering the compound or formulation described in the present application to prevent a disease or one or more symptoms associated with the disease, and includes: preventing the occurrence of the disease or disease state in a mammal, particularly when such a mammal is predisposed to the disease state but has not yet been diagnosed with it.

The term "therapeutically effective amount" refers to an amount of the compound of the present application for (i) treating or preventing a specific disease, condition or disorder; (ii) alleviating, ameliorating or eliminating one or more symptoms of a specific disease, condition or disorder, or (iii) preventing or delaying onset of one or more symptoms of a specific disease, condition or disorder described herein. The amount of the compound of the present application composing the "therapeutically effective amount" varies dependently on the compound, the disease state and its severity, the mode of administration, and the age of the mammal to be treated, but can be determined routinely by those skilled in the art in accordance with their knowledge and the present disclosure. Typically, the particle size is quantified by measuring the characteristic equivalent spherical diameter (referred to as the volume diameter) by laser diffraction, e.g., by using a laser particle size analyzer.

In the present application, the particle size distribution is expressed in terms of volume diameter (VD).

The term "D₅₀" refers to the particle size corresponding to a cumulative volume distribution percentage of 50%, which is referred to as the volume median diameter and physically means that particles with particle sizes less than it make up 50% of the total volume.

The term "D₉₀" refers to the particle size corresponding to a cumulative volume distribution percentage of 90%, which physically means that particles with particle sizes less than it make up 90% of the total volume.

### DETAILED DESCRIPTION

The following specific examples are intended to allow those skilled in the art to clearly understand and implement the present application. These specific examples should not be considered as limit to the scope of the present application, but merely as exemplary description and representative of the present application.

### Example 1

1) A formula amount of hydroxypropylcellulose was added to water¹. After complete dissolution, a compound of formula (II) was added with stirring and uniformly dispersed. The resulting mixture was sieved.
2) The suspension containing the compound of formula (II) above was subjected to high-pressure homogenization, with the particle size of the compound of formula (II) controlled at: D50 < 2 µm and D90 < 4 µm.
3) A 0.5 mol/L solution of maleic acid in water² was prepared. A formula amount of Eudragit^{®} E100 was dissolved in half of the prepared maleic acid solution. After complete dissolution, the resulting solution was added to the suspension obtained in step 2), and then the suspension was adjusted to a pH value between 3.0-3.5 with the rest of the maleic acid solution to obtain a suspension.
4) Sucrose blank pellet cores were coated with the suspension prepared in step 3) in a fluidized bed reactor to obtain drug-containing pellets.
5) The drug-containing pellets were well mixed in a hopper mixer.
6) Capsules were filled.

The specific composition of the pharmaceutical capsule formulation is shown in Table 1 below.

**Table 1**

| **Formula component** | **Amount (mg)** |
|---|---|
| Compound of formula (II) | 0.627 |
| Hydroxypropylcellulose (HPC-SL) | 0.10 |
| Sucrose | 3.13 |
| Eudragit^{®} E100 | 5 |
| Maleic acid | Proper amount |
| Sucrose pellet core (0.6-0.8 mm) | 333.33 |

| | |
|---|---|
| Note: water¹: 66.67 mg; and water²: 50 mg. | |

Examples 2-4 were prepared by referring to the procedures in Example 1. The specific composition of the pharmaceutical capsule formulations is shown in Table 2 below.

**Table 2**

| **Formula component** | **Amount (mg)** | | |
|---|---|---|---|
| | **Example 2** | **Example 3** | **Example 4** |
| Compound of formula (II) | 2.508 | 6.27 | 12.54 |
| Hydroxypropylcellulose (HPC-SL) | 0.40 | 1.0 | 2.0 |
| Sucrose | 12.54 | 31.35 | 62.7 |
| Eudragit^{®} E100 | 20 | 50 | 100 |
| Maleic acid | Proper amount | Proper amount | Proper amount |
| Sucrose pellet core (0.6-0.8 mm) | 250 | 250 | 250 |

| | | | |
|---|---|---|---|
| Note: Example 2, water¹: 266.68 mg; water²: 200 mg; Example 3, water¹: 666.7 mg; and water²: 500 mg; and Example 4, water¹: 1333.4 mg; and water²: 1000 mg. | | | |

### Example 5

The preparation was conducted by referring to steps 1)-6) of Example 1, except that in step 3), the pH value of the suspension was adjusted to 2.0 ≤ pH < 3.0; and the amount of water¹ was 25 mg, and the amount of water² is 50 mg.

The specific composition of the pharmaceutical capsule formulation is shown in Table 3 below.

**Table 3**

| **Formula component** | **Amount (mg)** |
|---|---|
| Compound of formula (II) | 2.508 |
| Hydroxypropylcellulose (HPC-SL) | 0.40 |
| Sucrose | 12.54 |
| Eudragit^{®} E100 | 20 |
| Maleic acid | Proper amount |
| Sucrose pellet core (0.6-0.8 mm) | 250 |

### Example 6

1) Hydroxypropylcellulose was added to water. After complete dissolution, a compound of formula (II) was added with stirring and uniformly dispersed. The resulting mixture was sieved.
2) The drug-containing suspension above was subjected to high-pressure homogenization.
3) A formula amount of sucrose was added to the treated suspension above and dissolved by stirring.
4) Sucrose blank pellet cores were coated with the drug suspension in a fluidized bed reactor to obtain drug-containing pellets.
5) The drug-containing pellets were well mixed in a hopper mixer.
6) Capsules were filled.

The specific composition of the pharmaceutical capsule formulation is shown in Table 4 below.

**Table 4**

| **Formula component** | **Amount (mg)** |
|---|---|
| Compound of formula (II) | 0.627 |
| Hydroxypropylcellulose (HPC-SL) | 0.10 |
| Sucrose | 3.13 |
| Sucrose pellet core (0.6-0.8 mm) | 333.33 |

### Example 7

1) A compound of formula (II) was mixed with a 9-fold amount of lactose. The mixture was jet-milled until the particle sizes D50 < 2 µm and D90 < 4 µm.
2) The milled mixture of the maleate salt of a compound of formula (I) and lactose, the rest of lactose, sodium carboxymethyl starch, talcum powder and silica were mixed in a high-energy mixer at a paddle speed of 400 rpm and a cutter speed of 1000 rpm for 5-10 min.
3) Capsules were filled.

The specific composition of the pharmaceutical capsule formulation is shown in Table 5 below.

**Table 5**

| **Formula component** | **Amount (mg)** |
|---|---|
| Compound of formula (II) | 0.625 |
| Lactose | 250.8 |
| Sodium carboxymethyl starch | 8.475 |
| Silicon dioxide | 1.1275 |
| Talcum powder | 5.65 |

### Experimental Example 1: Stability

### 1.1. Preparation of samples

The samples prepared in the above examples (a paper box was adopted for outer packaging; a composite hard sheet formed from polyamide/aluminum/polyvinyl chloride by cold stamping and aluminum foil for pharmaceutical packaging were adopted for inner packaging) were let stand in sample boxes under different conditions for stability testing (40 °C ± 2 °C and 75% ± 5% RH; 30 °C ± 2 °C and 65% ± 5% RH; 25 °C ± 2 °C and 60% ± 5% RH; and 20 °C ± 2 °C and 60% ± 5% RH) for 1 month, 2 months, 3 months or 6 months and taken for analysis to determine the change in the compound A (impurity) content.

### 1.2. Instrument

Instrument: SHIMADAZU LC-20AD HPLC with a Waters Xbridge Shield RP18 (150 × 4.6 mm, 3.5 µm) chromatography column or a column with equivalent performance.

0.01 mol/L ammonium formate buffer (0.63 g of ammonium formate was dissolved in 1000 mL of water, 1 mL of formic acid was added, and the pH value was adjusted to 7.4 with triethylamine) was used as mobile phase A, and acetonitrile as mobile phase B.

Linear gradient elution is performed according to the table below.

| Time (min) | Mobile phase A (%) | Mobile phase B (%) |
|---|---|---|
| 0 | 77 | 23 |
| 50 | 20 | 80 |
| 51 | 77 | 23 |
| 60 | 77 | 23 |

### 1.3. Preparation of sample solution

A proper amount of the capsule's contents of each of the samples above was added to a 20 mL measuring flask, and a solvent [acetonitrile-water (40:60)] was precisely added. The sample was ultrasonically dissolved, and the resulting mixture was well mixed by shaking, and then centrifuged. The supernatant was taken as a test solution. A proper amount of the test solution was precisely measured out and 100-fold diluted with the solvent to prepare a control solution.

Compound A had a retention time of 28-30 min, a relative retention time RRT of 1.3 (standard sample: the compound of formula (I)), and a peak at m/z 456.1600 ([M+2H]++) in a mass spectrum The results are shown in Tables 6-9 below.

**Table 6 (40 °C ± 2 °C and 75% ± 5% RH)**

| Example | Test item | 0 month (%) | 1 month (%) | 2 months (%) | 3 months (%) | 6 months (%) |
|---|---|---|---|---|---|---|
| Example 1 | Compound A | 0.07 | 0.12 | 0.14 | 0.15 | 0.16 |
| Example 2 | Compound A | 0.06 | 0.09 | 0.11 | 0.11 | 0.13 |
| Example 6 | Compound A | 0.06 | 0.47 | 0.62 | 0.78 | 1.24 |
| Example 7 | Compound A | Undetectable | 8.66 | / | / | / |

**Table 7 (30 °C ± 2 °C and 65% ± 5% RH)**

| Example | Test item | 0 month (%) | | 3 months (%) | 6 months (%) |
|---|---|---|---|---|---|
| Example 1 | Compound A | 0.07 | | 0.11 | 0.13 |
| Example 2 | Compound A | 0.06 | | 0.09 | 0.10 |
| Example 6 | Compound A | 0.06 | | 0.29 | 0.45 |

**Table 8 (25 °C ± 2 °C and 60% ± 5% RH)**

| Example | Test item | 0 month (%) | 3 months (%) | 6 months (%) |
|---|---|---|---|---|
| Example 1 | Compound A | 0.07 | 0.09 | 0.10 |
| Example 2 | Compound A | 0.06 | 0.07 | 0.08 |
| Example 6 | Compound A | 0.06 | 0.18 | 0.29 |
| Example 7 | Compound A | Undetectable | 1.63 | / |

**Table 9 (20 °C ± 2 °C and 60% ± 5% RH)**

| Example | Test item | 0 month (%) | 3 months (%) | 6 months (%) |
|---|---|---|---|---|
| Example 1 | Compound A | 0.07 | 0.08 | / |
| Example 2 | Compound A | 0.06 | / | 0.07 |
| Example 6 | Compound A | 0.06 | 0.13 | 0.19 |
| Example 7 | Compound A | Undetectable | 0.48 | / |

| | | | | |
|---|---|---|---|---|
| The "/" above indicates no detection. | | | | |

### Experimental Example 2: Dissolution Rate

The dissolution rates were measured by referring to the Method 2 (paddle, 0.1 mol/L hydrochloric acid medium, 900 mL, 75 rpm) of General Chapter 0931, *Chinese Pharmacopoeia,* Volume IV, 2015 Edition. The results are shown in Table 10.

**Table 10**

| Example | Dissolution rate |
|---|---|
| Example 1 | 100% |
| Example 2 | 103% |
| Example 6 | 93.7% |
| Example 7 | 90.7% |

## Claims

1. A solid pharmaceutical composition comprising a compound of formula (I) or a pharmaceutically acceptable salt thereof, and a polymethacrylate ester,

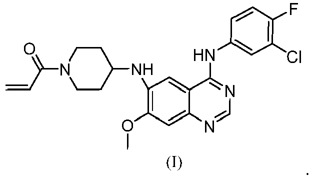

2. The solid pharmaceutical composition according to claim 1, wherein:
the pharmaceutically acceptable salt of the compound of formula (I) is selected from the group consisting of a maleate salt, a hydrochloride salt, a hydrobromide salt, a sulfate salt, a phosphate salt, a nitrate salt, an acetate salt, a lactate salt, a malonate salt, a succinate salt, a fumarate salt, a malate salt, a mandelate salt, a tartrate salt, a citrate salt, an ascorbate salt, a palmitate salt, a benzoate salt, a phenylacetate salt, a cinnamate salt, a salicylate salt, a methanesulfonate salt, a benzenesulfonate salt, and a methylbenzenesulfonate salt; optionally, the pharmaceutically acceptable salt is selected from the group consisting of a maleate salt, a malate salt, a fumarate salt, a tartrate salt, a citrate salt, a lactate salt, a phosphate salt, and an acetate salt; optionally, the pharmaceutically acceptable salt is selected from a maleate salt; or
a molar ratio of the compound of formula (I) to an acid radical ion in the pharmaceutically acceptable salt of the compound of formula (I) may be 1:1.

3. The solid pharmaceutical composition according to claim 2, wherein the pharmaceutically acceptable salt of the compound of formula (I) is a compound of formula (II),

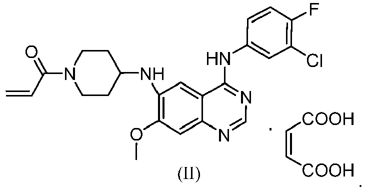

4. The solid pharmaceutical composition according to any one of claims 1-3, wherein:
the polymethacrylate ester is Eudragit^{®}; optionally, the polymethacrylate ester is selected from the group consisting of Eudragit^{®} E100, Eudragit^{®} EPO, Eudragit^{®} S100, Eudragit^{®} L100, Eudragit^{®} E100-55, Eudragit^{®} FS30D, Eudragit^{®} NM30D, Eudragit^{®} NE30D, Eudragit^{®} RL100, Eudragit^{®} RS100, and combinations thereof; optionally, the polymethacrylate ester is selected from Eudragit^{®} E100; or
wherein a weight ratio of the polymethacrylate ester to the compound of formula (I) or the pharmaceutically acceptable salt thereof is (50-0.5):1, or (45-1):1, or (40-1.5):1, or (35-2):1, or (30-3):1, or (25-4):1, or (20-5):1, or (18-5.5):1, or (16-5.7):1, or (14-5.9):1, or (12-6.1):1, or (10-6.3):1, or (8.5-6.5):1, or (8.5-7):1, or (8.5-7.5):1; or wherein a weight ratio of the polymethacrylate ester to the compound of formula (II) is 8:1.

5. The solid pharmaceutical composition according to any one of claims 1-4, wherein:
the solid pharmaceutical composition further comprises an acid; or,
the solid pharmaceutical composition further comprises an acid selected from the group consisting of: maleic acid, malic acid, fumaric acid, tartaric acid, citric acid, lactic acid, phosphoric acid, acetic acid, and a combination thereof; optionally, the acid is maleic acid.

6. The solid pharmaceutical composition according to any one of claims 1-5, wherein:
the solid pharmaceutical composition comprises 0.1-50 wt%, or 0.1-20 wt%, or 0.1-10 wt%, or 0.1-5 wt%, or 0.1-4 wt% of the compound of formula (I) or the pharmaceutically acceptable salt thereof based on the total mass of the solid pharmaceutical composition.

7. The solid pharmaceutical composition according to any one of claims 1-6, wherein:
the solid pharmaceutical composition further comprises a surface stabilizer; or
the solid pharmaceutical composition further comprises a surface stabilizer selected from the group consisting of hydroxypropylcellulose, lecithin, glycerol monostearate, polyoxyethylene castor oil derivatives, polyoxyethylene sorbitan fatty acid esters, sodium dodecyl sulfate, carboxymethylcellulose calcium, carboxymethylcellulose sodium, methylcellulose, hydroxyethylcellulose, hydroxypropylmethylcellulose, hydroxypropylmethylcellulose phthalate, amorphous cellulose, magnesium aluminum silicate, triethanolamine, polyvinyl alcohol, polyvinylpyrrolidone, 4-(1,1,3,3-tetramethylbutyl)phenol polymers with ethylene oxide and formaldehyde, and poloxamers; optionally, the surface stabilizer is selected from the group consisting of hydroxypropylcellulose, carboxymethylcellulose calcium, carboxymethylcellulose sodium, methylcellulose, hydroxyethylcellulose, hydroxypropylmethylcellulose, hydroxypropylmethylcellulose phthalate, and amorphous cellulose; optionally, the surface stabilizer is selected from hydroxypropylcellulose; or
the solid pharmaceutical composition further comprises a surface stabilizer in the following amount: a weight ratio of the surface stabilizer to the compound of formula (I) or the pharmaceutically acceptable salt thereof is (0.06-1):1, or (0.07-0.9):1, or (0.08-0.8):1, or (0.09-0.7):1, or (0.10-0.6):1, or (0.11-0.5):1, or (0.12-0.7):1, or (0.13-0.6):1, or (0.14-0.5):1, or (0.15-0.4):1, or (0.15-0.3):1, or (0.15-0.2):1; or wherein a weight ratio of the surface stabilizer to the compound of formula (II) is 0.16:1.

8. The solid pharmaceutical composition according to any one of claims 1-7, wherein:
the solid pharmaceutical composition further comprises a dispersant; or
the solid pharmaceutical composition further comprises a dispersant selected from the group consisting of:
sucrose, lactose, mannitol, or combinations thereof; optionally, the dispersant is selected from sucrose; or
the solid pharmaceutical composition further comprises a dispersant in the following amount: a weight ratio of the dispersant to the compound of formula (I) or the pharmaceutically acceptable salt thereof is (0.5-50):1, or (0.8-45):1, or (1.1-40):1, or (1.4-35):1, or (1.7-30):1, or (2-25):1, or (2.3-20):1, or (2.8-15):1, or (3.1-10):1, or (3.4-9):1, or (3.7-8):1, or (4.0-7):1, or (4.3-6):1, or (4.5-5.5):1; or wherein a weight ratio of the dispersant to the compound of formula (II) is 5:1.

9. The solid pharmaceutical composition according to any one of claims 1-8, wherein:
the solid pharmaceutical composition further comprises a carrier; or
the solid pharmaceutical composition further comprises a carrier selected from the group consisting of a cellulose sphere, a mannitol pellet core, a tartaric acid pellet core, a lactose/microcrystalline pellet core, a sucrose pellet core, a starch pellet core, and combinations thereof; optionally, the carrier is selected from the group consisting of a cellulose sphere and a sucrose pellet core; optionally, the carrier is selected from a sucrose pellet core; or
the solid pharmaceutical composition further comprises a carrier in the following amount: a proportion range of the carrier in the pharmaceutical composition is selected from the group consisting of 0.1-99 wt%, 0.5-99 wt%, 1-99 wt%, 5-99 wt%, 10-99 wt%, 15-99 wt%, 20-99 wt%, 25-99 wt%, 30-99 wt%, 35-99 wt%, 40-99 wt%, and 45-99 wt%.

10. The solid pharmaceutical composition according to any one of claims 1-4, comprising the compound of formula (I) or the pharmaceutically acceptable salt thereof, the polymethacrylate ester, an acid, a surface stabilizer, a dispersant, and a carrier.

11. The solid pharmaceutical composition according to claim 3, comprising the compound of formula (II), the polymethacrylate ester, an acid, a surface stabilizer, a dispersant, and a carrier.

12. The solid pharmaceutical composition according to claim 11, comprising the compound of formula (II), hydroxypropylcellulose, sucrose, the polymethacrylate ester, maleic acid, and a sucrose pellet core.

13. The solid pharmaceutical composition according to claim 11, comprising the following components in parts by weight:
| | |
|---|---|
| the compound of formula (II) | 0.1-40 parts |
| the surface stabilizer | 0.01-10 parts |
| the dispersant | 0.5-350 parts |
| the polymethacrylate ester the acid | 0.5-500 parts |
| the carrier | 100-500 parts; |
or, comprising the following components in parts by weight:
| | |
|---|---|
| the compound of formula (II) | 0.1-40 parts |
| hydroxypropylcellulose | 0.01-10 parts |
| sucrose | 0.5-350 parts |
| the polymethacrylate ester maleic acid | 0.5-500 parts |
| a sucrose pellet core | 100-500 parts; |
or, comprising the following components in parts by weight:
| | |
|---|---|
| the compound of formula (II) | 0.627 parts |
| hydroxypropylcellulose | 0.1 parts |
| sucrose | 3.13 parts |
| the polymethacrylate ester maleic acid | 5 parts |
| a sucrose pellet core | 333 parts; |
or, comprising the following components in parts by weight:
| | |
|---|---|
| the compound of formula (II) | 2.508 parts |
| hydroxypropylcellulose | 0.4 parts |
| sucrose | 12.54 parts |
| the polymethacrylate ester maleic acid | 20 parts |
| a sucrose pellet core | 250 parts; |
or, comprising the following components in parts by weight:
| | |
|---|---|
| the compound of formula (II) | 6.27 parts |
| hydroxypropylcellulose | 1 parts |
| sucrose | 31.35 parts |
| the polymethacrylate ester maleic acid | 50 parts |
| a sucrose pellet core | 250 parts; |
or, comprising the following components in parts by weight:
| | |
|---|---|
| the compound of formula (II) | 12.54 parts |
| hydroxypropylcellulose | 2 parts |
| sucrose | 62.7 parts |
| the polymethacrylate ester maleic acid | 100 parts |
| a sucrose pellet core | 250 parts. |

14. A method for preparing the solid pharmaceutical composition according to any one of claims 1-13, comprising the following steps:
preparing a suspension containing the compound of formula (I) or the pharmaceutically acceptable salt thereof;
adding the acid and the polymethacrylate ester to the suspension; and
subjecting the suspension to fluidized bed granulation, and more specifically, the fluidized bed granulation comprises loading the resulting suspension on the carrier on a fluidized bed to obtain drug-containing pellets.

15. Use of the solid pharmaceutical composition according to any one of claims 1-13 in the preparation a medicament for treating a tumor, and optionally, the tumor is selected from the group consisting of non-small cell lung cancer and breast cancer.
